# DEMANDE DE BREVET EUROPEEN

(11) **EP 0 965 306 A1**
(43) Date de publication de la demande: **22.12.1999**
(21) Numéro de dépôt: 99401501.4
(22) Date de dépôt: 17.06.1999
(51) Int. Cl.: A61B 5/09, A61B 5/087

(54) **Dispositif de détermination de phases respiratoires du sommeil d'un utilisateur**

(30) Priorité: 18.06.1998 FR 9807695
(71) Demandeur: TAEMA, F-92182 Antony Cédex (FR)
(72) Inventeur: Ruton, Stéphane, 78220 Viroflay (FR); Jonquet, Benoit, 78180 Montigny le Bretonneux (FR)
(74) Mandataire: Le Moenner, Gabriel

(57) **Abrégé**

L'invention concerne un dispositif (1) de détermination de phases respiratoires du sommeil d'un utilisateur (3), comprenant des moyens (5) de mesure d'au moins deux variables physiques dont au moins une première variable physique est représentative du flux nasal de l'utilisateur (3) et dont au moins une seconde variable physique est représentative du flux buccal de celui-ci. Le dispositif comprend en outre des moyens (21) de traitement et de conversion de chaque variable physique en vue d'établir son degré d'appartenance à au moins un état d'une variable floue, et des moyens (23) d'application de règles préétablies entre au moins un état d'une première variable floue et un état d'une seconde variable floue pour évaluer le degré d'appartenance à un état de phase respiratoire du sommeil de l'utilisateur (3) selon la logique floue.

## Description

L'invention est relative à un dispositif et à un procédé de détermination de phases respiratoires du sommeil d'un utilisateur.

Les troubles respiratoires du sommeil, tel le Syndrome d'Apnée du Sommeil (SAS) se caractérisent, en général, par un dysfonctionnement de la fonction respiratoire au cours du sommeil.

On observe chez les sujets victimes d'un tel syndrome une fragmentation du sommeil importante avec des phases de sommeil courtes et la reprise d'une respiration normale s'accompagnant le plus souvent d'un éveil bref, de quelques secondes.

Le déroulement normal du sommeil, du stade de sommeil léger au stade de sommeil profond, en passant par un stade de sommeil paradoxal, est fortement perturbé, ce qui a des conséquences sur la vie diurne de ces sujets. Ceux-ci ont, en effet, tendance à être somnolents, voire à s'endormir en pleine activité, et on observe également chez eux une diminution des fonctions intellectuelles et sexuelles, et des risques d'hypertension et d'insuffisance cardiaque.

Les troubles respiratoires peuvent être de types obstructifs ou centraux.

Dans le premier cas, on observe une obstruction totale (apnée obstructive) ou partielle (hypopnée obstructive) des voies aériennes supérieures alors que l'effort musculaire est maintenu. Ce type de troubles est souvent associé à un ronflement important.

Dans le second cas, la commande musculaire est absente (apnée centrale) ou diminuée (hypopnée centrale), les voies aériennes supérieures étant ouvertes.

Les troubles obstructifs représentent la grande majorité des troubles respiratoires nocturnes.

Habituellement, le diagnostic des troubles respiratoires du sommeil est effectué dans un laboratoire du sommeil grâce à l'enregistrement et l'étude de nombreux paramètres liés :
- soit au sommeil: électroencéphalogramme, électroocculogramme, électro-myogramme,
- soit à la fonction cardio-respiratoire : électrocardiogramme, fréquence respiratoire, flux nasal et/ou buccal, mouvements thoraciques et abdominaux, saturation artérielle en oxygène, ronflements.

Le diagnostic de ces pathologies du sommeil est récent et sa mise en oeuvre est lourde et nécessite l'hospitalisation du sujet pendant une ou deux nuits.

Le traitement des troubles respiratoires du sommeil met en oeuvre, habituellement, un appareillage permettant de maintenir les voies aériennes supérieures ouvertes.

Cet appareillage comporte le plus souvent un appareil de pression positive au masque (CPAP), dans lequel un moyen de compression de l'air ambiant, piloté par un dispositif de commande, délivre dans un conduit puis dans un masque placé sur le nez du patient de façon étanche, une pression d'air.

Le dispositif de commande compare la valeur de la pression dans le masque à une valeur de consigne de pression réglée et, selon l'écart mesuré, ajuste un signal de commande qu'il délivre au moyen de la compression de l'air ambiant.

Actuellement, la valeur de consigne de pression à appliquer dans le masque du patient est déterminée empiriquement dans le laboratoire du sommeil par augmentation progressive d'une valeur de consigne initiale et observation des conséquences sur les troubles respiratoires, la valeur choisie étant la première valeur pour laquelle on observe un sommeil normal non fragmenté.

II existe des appareils de diagnostic ou/et de traitement de ces troubles respiratoires nocturnes, détectant les apnées ou les hypopnées, mais ne différenciant pas les événements centraux et obstructifs.

D'autres, après avoir détecté une apnée, envoient dans le masque du patient une impulsion de pression et étudient l'écho éventuel : s'il n'y a pas d'écho, l'événement est central, et si un écho revient, l'événement est obstructif.

De tels appareils sont notamment décrits dans les documents FR-A-2663547 et FR-A-2674133.

Toutefois, afin de pouvoir établir un diagnostic correct des troubles respiratoires (apnées, hypopnées, pathologie de la résistance augmentée...) et/ou de déterminer et/ou mettre en place un traitement adéquat et efficace, il peut être nécessaire, voire indispensable, que le praticien, c'est-à-dire le médecin ou analogue, dispose d'une image fiable représentative de la succession des différentes phases inspiratoires et expiratoires de son patient, c'est-à-dire de l'image du débit ou flux respiratoire du sujet.

Cependant, les procédés et appareils existants ne permettent pas une telle détermination fiable et précise de l'image dudit débit respiratoire.

Dès lors, on comprend aisément, que le diagnostic et/ou le traitement subséquent peuvent être incorrects ou incomplets.

La présente invention a donc pour buts de proposer un procédé et un appareil de diagnostic et/ou de traitement des troubles respiratoires du sommeil :
- susceptibles de permettre la détermination fiable et précise, à partir de divers paramètres de respiration d'un patient, des diverses phases respiratoires de celui-ci,
- susceptibles de déterminer avec précision les phases de ronflement et / ou des phénomènes avec une obstruction partielle des voies respiratoires du patient,
- utilisables tant dans un laboratoire du sommeil, c'est-à-dire en milieu hospitalier, qu'au domicile du patient, et
- ayant un coût raisonnable.

A cet effet, l'invention a pour objet un dispositif de détermination de phases respiratoires du sommeil d'un utilisateur, comprenant des moyens de mesure d'au moins deux variables physiques dont au moins une première variable physique est représentative du flux nasal de l'utilisateur et dont au moins une seconde variable physique est représentative du flux buccal de celui-ci, caractérisé en ce qu'il comprend en outre des moyens de traitement et de conversion de chaque variable physique en vue d'établir son degré d'appartenance à au moins un état d'une variable floue, et des moyens d'application de règles préétablies entre au moins un état d'une première variable floue et un état d'une seconde variable floue pour évaluer le degré d'appartenance à un état de phase respiratoire du sommeil de l'utilisateur selon la logique floue.

Le dispositif selon l'invention peut de plus comporter une ou plusieurs des caractéristiques suivantes :
- chaque variable floue possède au moins deux états,
- lesdits états des phases respiratoires du sommeil comprennent au moins un état de respiration normale, un état d'apnée et un état d'hypopnée,
- les degrés d'appartenance des états associés à une variable floue sont établis à partir de courbes continues définies sur tout l'univers de discours d'une variable physique mesurée,
- les moyens de mesure comprennent un capteur de pression relié à une pièce nasale destinée à être portée par l'utilisateur et une des variables physiques mesurées est le signal de pression mesuré par ledit capteur de pression,
- lesdits moyens de traitement et de conversion comprennent des moyens d'extraction du signal de pression mesuré de phases de ronflement associées à des phénomènes obstructifs de respiration,
- lesdits moyens d'extraction de phases de ronflement comprennent des moyens de filtrage passe-haut du signal de pression, des moyens d'amplification du signal filtré, des moyens d'interpolation dudit signal filtré amplifié pour obtenir une courbe-enveloppe, des moyens de mémorisation d'une courbe de référence et des moyens de comparaison de ladite courbe-enveloppe avec ladite courbe de référence pour déterminer la présence de phases de ronflement,
- les moyens de mesure comprennent un capteur de courant mesurant le courant consommé par une turbine à faible inertie qui est reliée à ladite pièce nasale, et une des variables physiques mesurées est le courant consommé par ladite turbine,
- les moyens de traitement et de conversion comprennent des moyens pour extraire du signal de courant consommé une image du flux nasal de l'utilisateur, des moyens de détermination des phases d'inspiration et d'expiration nasales dudit utilisateur, des moyens de calcul de la dérivée temporelle de l'amplitude du flux nasal lors d'une phase d'inspiration et des moyens d'exploitation de la dérivée pour déterminer si l'utilisateur souffre d'un phénomène obstructif partiel ou pas,
- lesdits moyens d'exploitation de la dérivée de l'amplitude du flux nasal comprennent des moyens de comparaison de la valeur absolue de la dérivée à au moins une valeur de référence, et des moyens de mesure de la durée pendant laquelle la valeur absolue de la dérivée est inférieure à ladite au moins une valeur de référence,
- lesdits moyens d'exploitation de la dérivée comprennent en outre des moyens de relevé du nombre de changements de signe de la dérivée lorsque celle-ci est inférieure à la valeur de référence,
- les moyens de mesure comprennent un organe de mesure de la résistance d'une thermistance destinée à être placée à proximité de la bouche dudit utilisateur, et une des variables physiques mesurées est la résistance mesurée par ledit organe de mesure de la résistance de la thermistance.

L'invention a également pour objet un procédé de détermination de phases respiratoires du sommeil d'un utilisateur, caractérisé en ce qu'il comprend les étapes suivantes :
- on mesure au moins deux variables physiques dont au moins une première variable physique est représentative du flux nasal de l'utilisateur et dont au moins une seconde variable physique est représentative du flux buccal de celui-ci,
- on traite et convertit chaque variable physique en vue d'établir son degré d'appartenance à au moins un état d'une variable floue, et
- on applique des règles préétablies entre au moins un état d'une première variable floue et un état d'une seconde variable floue pour évaluer le degré d'appartenance à un état de phase respiratoire du sommeil de l'utilisateur selon la logique floue.

Ce procédé peut de plus comporter une ou plusieurs des caractéristiques suivantes :
- chaque variable floue possède au moins deux états,
- lesdits états des phases respiratoires du sommeil comprennent au moins un état de respiration normale, un état d'apnée et un état d'hypopnée,
- on établit les degrés d'appartenance des états associés à une variable floue à partir de courbes continues définies sur tout l'univers de discours d'une variable physique mesurée,
- une des variables physiques mesurées est la pression dans une pièce nasale portée par l'utilisateur,
- lors du traitement et de la conversion, on extrait du signal de pression mesuré des phases de ronflement associées à des phénomènes obstructifs de respiration,
- lors de l'extraction, on réalise un filtrage passe-haut du signal de pression, on amplifie le signal filtré, on interpole ledit signal filtré amplifié pour obtenir une courbe-enveloppe, et on compare ladite courbe-enveloppe à une courbe de référence pour déterminer la présence de phases de ronflement,
- pour obtenir une image du flux nasal, on mesure par exemple le courant consommé par une turbine à faible inertie qui est reliée à ladite pièce nasale portée par l'utilisateur,
- lors de l'extraction de ladite image du flux nasal, on détermine les phases d'inspiration et d'expiration dudit utilisateur et on calcule la dérivée temporelle de l'amplitude du flux nasal lors d'une phase d'inspiration et on exploite cette dérivée ainsi calculée pour déterminer si l'utilisateur souffre d'un phénomène obstructif partiel ou pas,
- lors de l'exploitation de la dérivée de l'amplitude du flux nasal, on compare la valeur absolue de cette dérivée à au moins une valeur de référence, et on mesure la durée pendant laquelle la valeur absolue de la dérivée est inférieure à ladite au moins une valeur de référence,
- lors de l'exploitation de la dérivée de l'amplitude du flux nasal, on relève en outre le nombre de changements de signe de la dérivée lorsque la valeur absolue de celle-ci est inférieure à ladite au moins une valeur de référence,
- pour obtenir une image du flux buccal, on mesure par exemple la résistance d'une thermistance placée à proximité de la bouche dudit utilisateur.

L'invention a également pour objet une méthode de diagnostic des phases respiratoires du sommeil d'un patient souffrant de troubles respiratoires de sommeil, en particulier de l'apnée du sommeil, caractérisé en ce qu'il comprend les étapes suivantes :
- on mesure au moins deux variables physiques dont au moins une première variable physique est représentative du flux nasal de l'utilisateur et dont au moins une seconde variable physique est représentative du flux buccal de celui-ci,
- on traite et convertit chaque variable physique en vue d'établir son degré d'appartenance à au moins un état d'une variable floue, et
- on applique des règles préétablies entre au moins un état d'une première variable floue et un état d'une seconde variable floue pour évaluer le degré d'appartenance à un état de phase respiratoire du sommeil du patient selon la logique floue.

Ce procédé peut de plus comporter une ou plusieurs des caractéristiques suivantes :
- chaque variable floue possède au moins deux états,
- lesdits états des phases respiratoires du sommeil comprennent au moins un état de respiration normale, un état d'apnée et un état d'hypopnée,
- on établit les degrés d'appartenance des états associés à une variable floue à partir de courbes continues définies sur tout l'univers de discours d'une variable physique mesurée,
- une des variables physiques mesurées est la pression dans une pièce nasale portée par l'utilisateur,
- lors du traitement et de la conversion, on extrait du signal de pression mesuré des phases de ronflement associées à des phénomènes obstructifs de respiration,
- lors de l'extraction, on réalise un filtrage passe-haut du signal de pression, on amplifie le signal filtré, on interpole ledit signal filtré amplifié pour obtenir une courbe-enveloppe, et on compare ladite courbe-enveloppe à une courbe de référence pour déterminer la présence de phases de ronflement,
- pour obtenir une image du flux nasal, on mesure par exemple le courant consommé par une turbine à faible inertie qui est reliée à ladite pièce nasale portée par le patient,
- lors de l'extraction de ladite image du flux nasal, on détermine les phases d'inspiration et d'expiration dudit patient et on calcule la dérivée temporelle de l'amplitude du flux nasal lors d'une phase d'inspiration et on exploite cette dérivée ainsi calculée pour déterminer si le patient souffre d'un phénomène obstructif partiel ou pas,
- lors de l'exploitation de la dérivée de l'amplitude du flux nasal, on compare la valeur absolue de cette dérivée à au moins une valeur de référence, et on mesure la durée pendant laquelle la valeur absolue de la dérivée est inférieure à ladite au moins une valeur de référence,
- lors de l'exploitation de la dérivée de l'amplitude du flux nasal, on relève en outre le nombre de changements de signe de la dérivée lorsque la valeur absolue de celle-ci est inférieure à ladite au moins une valeur de référence,
- pour obtenir une image du flux buccal, on mesure par exemple la résistance d'une thermistance placée à proximité de la bouche dudit patient.

D'autres caractéristiques et avantages de l'invention ressortiront de la description suivante, donnée à titre d'exemple, sans caractère limitatif, en regard des dessins annexés sur lesquels :
La figure 1 est un schéma synoptique d'un dispositif selon l'invention,
La figure 2 est un organigramme montrant certaines étapes de traitement d'une variable physique représentative du flux nasal d'un utilisateur,
La figure 3 montre deux graphiques pour illustrer en particulier les étapes décrites en référence à la figure 2,
La figure 4 représente un graphique montrant un flux nasal en fonction du temps dans le cas où les voies respiratoires de l'utilisateur sont affectées par une obstruction partielle,
La figure 5 représente un graphique montrant la dérivée temporelle du flux nasal de la figure 4,
La figure 6 montre deux graphiques pour illustrer la détermination d'un phénomène de ronflement à partir d'un signal de pression mesuré au niveau de la pièce nasale et / ou buccale appliquée sur l'utilisateur, et
La figure 7 montre un graphique pour illustrer la conversion d'une variable physique mesurée en vue d'établir son degrés d'appartenance à un ou plusieurs états d'une variable floue.

### I. Structure du dispositif selon l'invention

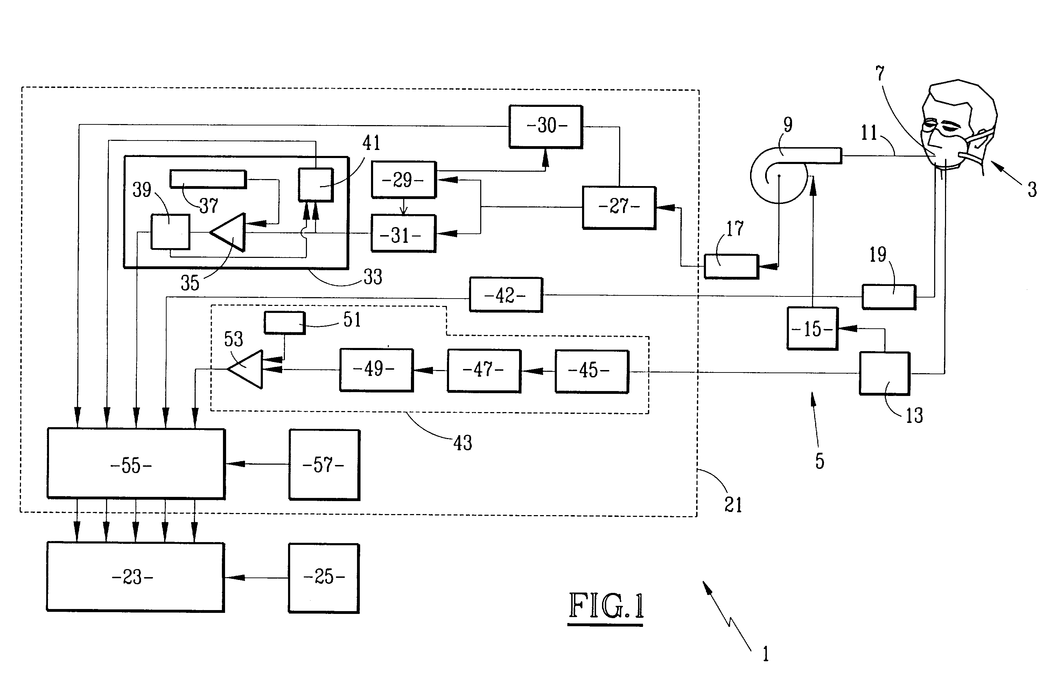

Sur la figure 1 est représenté un dispositif 1 selon l'invention permettant de déterminer des phases respiratoires du sommeil d'un utilisateur 3.

Ce dispositif 1 comprend des moyens de mesure 5 d'au moins deux variables physiques dont au moins une première variable physique est représentative du flux nasal de l'utilisateur et dont au moins une seconde variable physique est représentative du flux buccal de celui-ci.

A cet effet, l'utilisateur 3 porte une pièce buccale et/ou nasale 7, tel qu'un masque et/ou des lunettes respiratoires qui sont connus en soi et ne seront pas décrits en détail ici.

A cette pièce nasale/ buccale 7 est raccordée une turbine 9 de faible inertie au moyen d'un conduit respiratoire 11 permettant d'acheminer le gaz respiratoire sous pression jusqu'aux voies respiratoires de l'utilisateur 3.

Selon le cas, le gaz respiratoire est distribué à une pression positive constante au cours du temps, c'est-à-dire à un seul niveau de pression (appareil de type CPAP), ou à une pression variant entre au moins un niveau de pression basse et au moins un niveau de pression plus élevée, c'est-à-dire à plusieurs niveaux de pression (appareil de type BPAP). Le fonctionnement de tels appareils ayant déjà été décrit à maintes reprises dans l'art antérieur, celui-ci ne sera pas détaillé ci-après. Toutefois, pour plus de détails, on pourra se reporter notamment aux documents suivants: US-A-5 492 113, US-A-5 239 995, EP-A-0 656 216 ou EP-A-0 505 232.

Un capteur de pression 13 dont le point de prise de pression est agencé sur la pièce nasale/buccale 7, c'est-à-dire à proximité immédiate des voies respiratoires de l'utilisateur 3, permet de détecter les variations de pression dues à la respiration de celui-ci. Ce capteur 13 est relié à des moyens 15 de commande de la turbine 9 pour fournir à ces moyens 15 un signal de pression. En fonction du signal de pression reçu et d'une valeur de consigne de surpression déterminée, ces moyens 15 fournissent un signal de commande à la turbine 9 de manière à moduler la surpression fournie par la turbine 9 à l'utilisateur 3 (voir aussi EP-A-505232 ou US-A-5,443,061).

Etant donné que la pression du gaz respiratoire fourni à l'utilisateur doit être quasiment constante, on comprend que le régime moteur de ladite turbine 9 et donc la quantité de courant consommée par celle-ci varie en fonction du signal de commande des moyens 15 et correspond donc au flux nasal de l'utilisateur 3.

C'est pourquoi, pour obtenir une image du flux nasal, un capteur de courant 17 mesure le courant consommé par la turbine 9.

De plus, afin de compléter l'image du flux nasal par une image du flux buccal, un capteur comme par exemple une thermistance (non visible sur la figure) est placé dans la pièce buccale / nasale à proximité immédiate de la bouche de l'utilisateur 3.

Ce capteur, par exemple la thermistance mentionnée ci-dessus, est relié à un organe 19 de mesure mesurant dans le cas d'une thermistance la résistance de celle-ci.

En effet, la valeur de mesure comme par exemple la résistance de la thermistance se trouve modifiée par des variations de température provoquées par un flux buccal éventuel sortant de la bouche de l'utilisateur 3, ce qui permet d'obtenir une image du flux buccal fiable et précis.

Pour déterminer les phases respiratoires du sommeil de l'utilisateur 3 à partir des valeurs des variables physiques mesurées par l'intermédiaire des capteurs 13, 19 et 17, c'est-à-dire les images de la pression, du flux nasal et du flux buccal, le dispositif 1 comprend en outre des moyens 21 de traitement et de conversion de chaque variable physique relevée en vue d'établir son un degré d'appartenance à au moins un état d'une variable floue. Les degrés d'appartenance des états respectifs des variables floues sont introduits dans des moyens 23 d'application de règles mémorisées dans une mémoire 25 formant une base de connaissances.

Les moyens 21 de traitement et de conversion comprennent des moyens 27 pour extraire du signal de courant consommé, mesuré par le capteur de courant 17, une image du flux nasal de l'utilisateur 3, c'est-à-dire la variation du flux nasal dans le temps. Cette image du flux nasal de l'utilisateur 3 est introduite dans des moyens 29 de détermination des phases d'inspiration et d'expiration nasale de l'utilisateur, dans des moyens 30 de détermination de l'amplitude du flux nasal et dans des moyens 31 de calcul de la dérivée temporelle de l'amplitude du flux nasal.

Les moyens 30 reçoivent en outre un signal de commande des moyens 29 indiquant la phase de respiration dans laquelle se trouve l'utilisateur.

Les moyens 31 ne calculent la dérivée du flux nasal que lors d'une phase d'inspiration. C'est pourquoi les moyens 29 envoient également un signal de commande aux moyens 31 de calcul de la dérivée lorsqu'ils ont déterminé que l'utilisateur est dans une phase d'inspiration nasale.

La dérivée calculée par les moyens 31 est introduite dans des moyens d'exploitation 33. Ces moyens d'exploitation 33 comprennent des moyens 35 de comparaison de la valeur absolue de la dérivée à au moins une valeur de référence enregistrée dans une mémoire 37. En fonction du résultat de comparaison, les moyens 35 activent des moyens 39 de mesure de la durée pendant laquelle la valeur absolue de la dérivée est inférieure à ladite au moins une valeur de référence.

De plus, les moyens 33 comprennent, reliés aux moyens 31 de calcul de la dérivée ainsi qu'aux moyens 35 de comparaison, des moyens 41 de relevé du nombre de changements de signe de la dérivée lorsque la valeur absolue de celle-ci est inférieure à la valeur de référence.

Par l'intermédiaire des moyens 33 d'exploitation de la dérivée de l'amplitude du flux nasal, on détermine, si l'utilisateur souffre d'une obstruction partielle ou pas des voies respiratoires, comme cela sera expliqué en détail ci-après.

Les moyens 21 de traitement et de conversion comprennent en outre des moyens 42 de traitement du signal délivré par l'organe 19 de mesure par exemple de la résistance de la thermistance.

Par ailleurs, les moyens 21 de traitement et de conversion comprennent des moyens 43 d'extraction du signal de pression, mesuré par le capteur de pression 13, de phases de ronflement associées à des phénomènes obstructifs de respiration.

Ces moyens 43 d'extraction comprennent des moyens 45 de filtrage passe-haut du signal de pression, des moyens 47 d'amplification du signal filtré, des moyens 49 d'interpolation dudit signal filtré amplifié pour obtenir une courbe-enveloppe, des moyens 51 de mémorisation d'une courbe de référence et des moyens 53 de comparaison de ladite courbe-enveloppe avec ladite courbe de référence pour déterminer la présence de phases de ronflement.

Les diverses variables physiques ainsi traitées sont introduites dans des moyens 55 de conversion de chacune de ces variables en vue d'établir son degré d'appartenance à au moins un état d'une variable floue associée. Cette conversion qui sera expliquée plus en détail ci-après, est réalisée à partir de courbes continues définies sur tout l'univers de discours des variables physiques et mémorisées dans une base de données 57 reliée aux moyens de conversion 55.

### II. Fonctionnement du dispositif selon l'invention

On expliquera ci après le fonctionnement du dispositif 1 selon l'invention en exposant en détail d'une part les diverses étapes de traitement et de conversion mises en oeuvre dans les moyens 21 de traitement et de conversion, et d'autre part la mise en oeuvre de l'application des règles aux variables floues par les moyens 23 selon la logique floue.

### II.1 Détermination des phases d'inspiration et d'expiration nasales de l'utilisateur ainsi que de l'amplitude du flux nasal

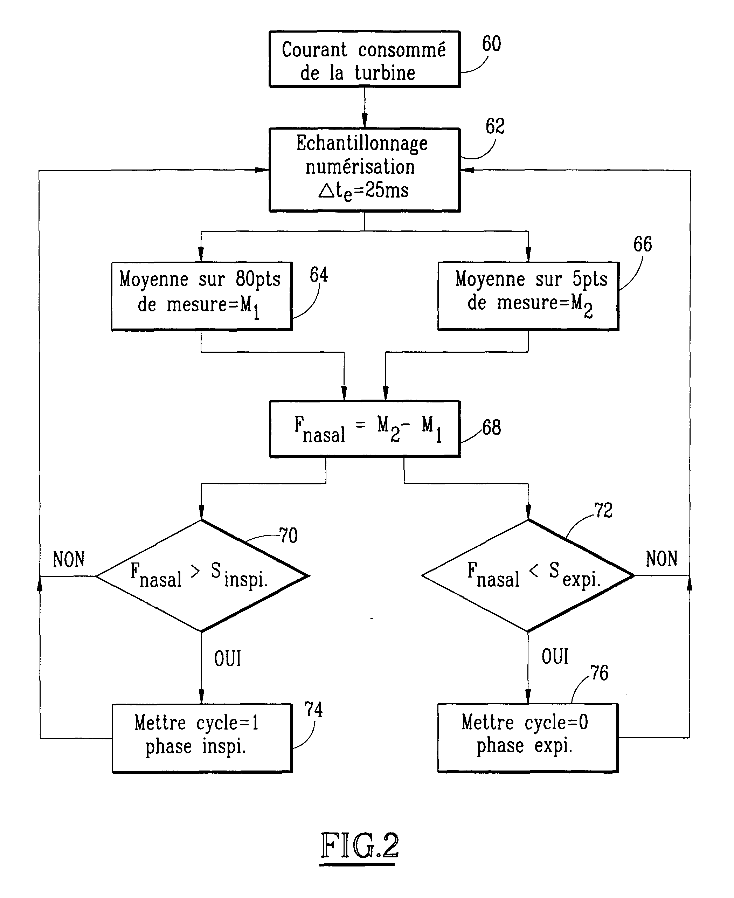

Sur la figure 2 sont représentées les principales étapes de fonctionnement mises en oeuvre par les moyens 27 et 29.

Lors d'une première étape 60, le signal de courant consommé par la turbine 9, délivré par le capteur de courant 17, est introduit dans les moyens 27 d'extraction et y est ensuite numérisé et échantillonné à une cadence de Δtₑ = 25ms lors d'une seconde étape 62.

Ensuite, lors d'une étape 64, on calcule la moyenne M₁ de quatre-vingt valeurs échantillonnées, ce qui correspond à une moyenne des valeurs de mesure sur une durée de 20 secondes. Cette durée de 20s correspond à deux ou trois cycles respiratoires de l'utilisateur.

En parallèle, au cours d'une étape 66, on calcule la moyenne M2 de cinq valeurs échantillonnées, ce qui correspond à une moyenne des valeurs de mesure sur une durée de 125 millisecondes et est en fait le signal quasiment brut, légèrement lissé.

Lors d'une étape suivante 68, on calcule le flux nasal Fₙₐₛₐₗ comme étant la différence entre M₂ et M₁.

Puis, pendant des étapes 70 et 72, Fₙₐₛₐₗ est comparé à des seuils respectifs Sᵢₙₛₚᵢ et Sₑₓₚᵢ. Les seuils Sᵢₙₛₚᵢ et Sₑₓₚᵢ correspondent à des valeurs représentatives du débit du flux nasal respectivement au dessus ou en dessous desquels il est quasiment certain que l'utilisateur est dans une phase d'inspiration ou d'expiration. Bien entendu, Sᵢₙₛₚᵢ est supérieur à Sₑₓₚᵢ. Les seuils Sᵢₙₛₚᵢ et Sₑₓₚᵢ peuvent être déterminés empiriquement par des essais cliniques sur des utilisateurs.

Si, lors de l'étape 70, Fₙₐₛₐₗ est supérieur au seuil Sᵢₙₛₚᵢ, la valeur une est attribuée lors d'une étape 74 à une variable nommée cycle, cycle = 1 signifiant donc que l'utilisateur 3 se trouve dans une phase d'inspiration nasale, et ensuite on revient à l'étape 62. Si Fₙₐₛₐₗ est inférieur à la valeur de seuil Sᵢₙₛₚᵢ, on revient directement à l'étape 62.

Si, pendant l'étape de comparaison 72, Fₙₐₛₐₗ est inférieur à Sₑₓₚᵢ, on attribue lors de l'étape 76 la valeur zéro à la variable cycle, cycle = 0 signifiant que l'utilisateur 3 se trouve dans une phase d'expiration nasale, et ensuite on revient à l'étape 62. Si Fₙₐₛₐₗ est supérieur à la valeur de seuil Sₑₓₚᵢ, on revient directement à l'étape 62.

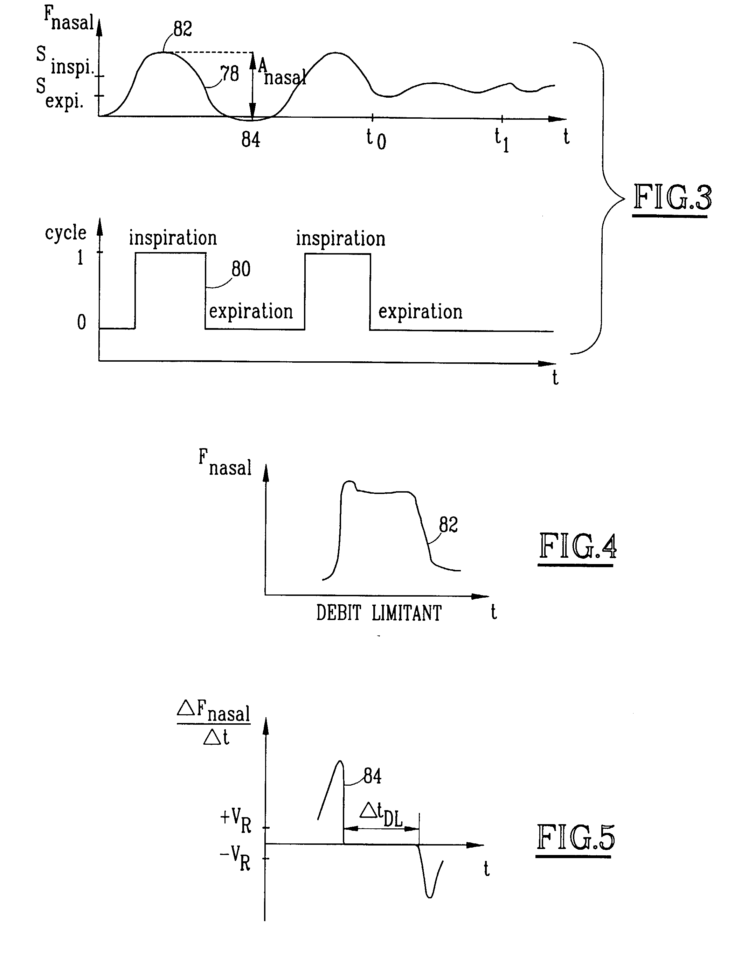

Ces étapes permettant de déterminer les phases d'inspiration et d'expiration nasale de l'utilisateur sont illustrées sur la figure 3. Cette figure 3 montre deux graphiques dont l'un, le graphique supérieur, montre une courbe 78 du flux nasal Fₙₐₛₐₗ en fonction du temps et l'autre, le graphique inférieur, montre une courbe 80 des valeurs de la variable cycle attribuées à cette variable en fonction de la valeur du flux nasal Fₙₐₛₐₗ.

Sur cette figure, on voit clairement que dès que Fₙₐₛₐₗ dépasse le seuil Sᵢₙₛₚᵢ, cycle prend la valeur 1, et si Fₙₐₛₐₗ est inférieur à Sₑₓₚᵢ cycle prend la valeur 0. Jusqu'à l'instant référencé t₀, on peut considérer que l'utilisateur possède un cycle de respiration normale.

Au delà de t₀, Fₙₐₛₐₗ n'arrive plus, après l'entrée dans une phase d'expiration, à dépasser Sᵢₙₛₚᵢ et cycle reste à la valeur 0. Ceci peut être dû à une respiration buccale de l'utilisateur, mais aussi à une hypopnée ou une apnée du sommeil.

En outre, en mesurant le temps entre le début de deux phases d'inspiration (cycle=1), on détermine la durée des cycles de respiration de l'utilisateur. En mesurant la durée des phases d'expiration, on détermine les plages potentielles d'hypopnée et d'apnée de sommeil, car on considère que ces phénomènes ne se produisent que pour des phases prolongées d'expiration nasales, typiquement pour des phases d'expiration ayant une durée supérieure à 3 secondes. Par exemple sur la figure 3, l'utilisateur se trouve à partir de l'instant t₀ dans une phase d'expiration nasale. A partir de l'instant t₁, postérieur de 3s de t₀, l'utilisateur se trouve encore dans la phase d'expiration nasale et donc dans une plage de temps potentielle d'hypopnée ou d'apnée de sommeil.

Par ailleurs, pour calculer l'amplitude du flux nasal Aₙₐₛₐₗ, les moyens 30 déterminent lors d'une phase d'inspiration le maximum du flux nasal Fₙₐₛₐₗ (portant le numéro de référence 82) et lors d'une phase d'expiration le minimum du flux nasal Fₙₐₛₐₗ (portant le numéro de référence 84), et calculent la différence entre ces maximum et minimum.

Lors d'une phase d'expiration prolongée, c'est-à-dire ayant par exemple une durée supérieure à 3 secondes, les moyens 30 reçoivent des moyens 29 un signal de commande pour calculer l'amplitude du flux nasal à chaque seconde, c'est-à-dire pour calculer la différence entre le flux nasal déterminé à un instant donnée et celui à un instant lui précédent par exemple d'une seconde. Sur la figure 3, cette détermination à des intervalles de temps rapprochés de l'amplitude du flux nasal est mise en oeuvre par les moyens 30 à partir de l'instant t₁.

### II.2 Détermination d'un événement respiratoire obstructif dit de "débit limitant"

Un cycle de respiration normale possède une forme sinusoïdale comme cela est représenté sur la figure 3 jusqu'à l'instant t₀. Dans le cas d'une obstruction partielle des voies respiratoires de l'utilisateur, le flux inspiratoire qui augmente en début d'une phase d'inspiration, est rapidement limité de sorte que la courbe Fₙₐₛₐₗ possède une forme écrêtée. Une telle courbe 82 de Fₙₐₛₐₗ en fonction du temps dans le cas d'un débit limitant est représentée à titre d'exemple sur le graphique de la figure 4.

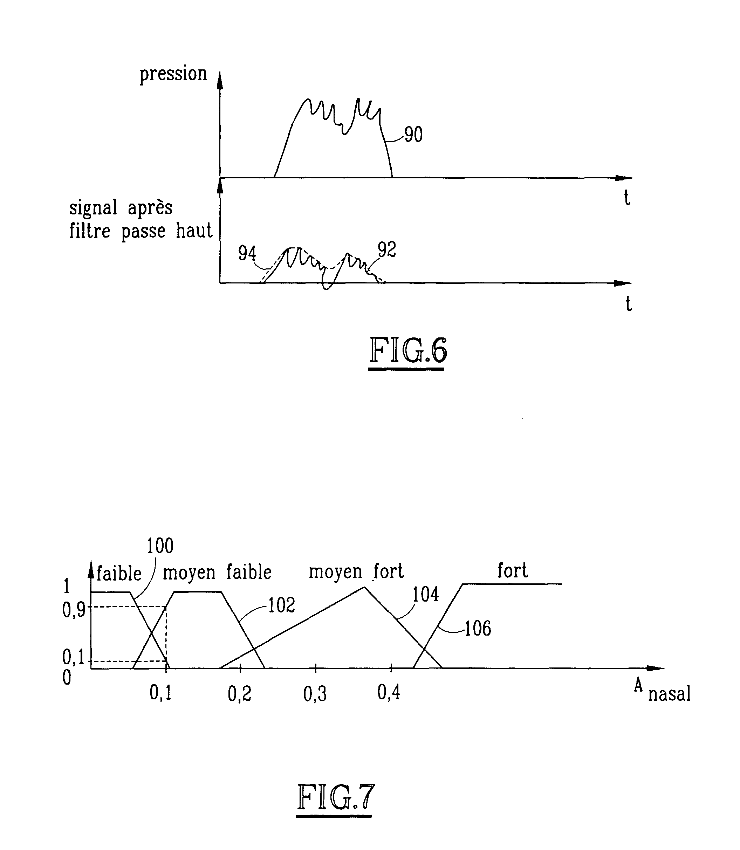

Afin de pouvoir détecter avec précision un tel phénomène, les moyens 31 calculent, lors d'une phase d'inspiration, la dérivée temporelle de l'amplitude du flux nasal Fₙₐₛₐₗ représentée par la courbe 84 de la figure 5.

La valeur absolue de la dérivée temporelle ainsi calculée est comparée à chaque instant par les moyens 35 à une valeur de référence V_{R} enregistrée dans la mémoire 37. Bien entendu, on peut également prévoir plusieurs valeurs de référence si cela s'avère nécessaire.

En outre, commandés par les moyens 35 de comparaison, les moyens 39 mesurent la durée Δt_{DL} pendant laquelle la valeur absolue de la dérivée est inférieure à la valeur de référence V_{R}.

Par ailleurs, pendant Δt_{DL} les moyens 41 relèvent le nombre de changements de signe de la dérivée.

Du moment que Δt_{DL} est supérieur à une durée de seuil comprise par exemple entre une et deux secondes, on peut conclure que l'utilisateur souffre d'un phénomène obstructif partiel. Ce diagnostic est renforcé lorsque le signe de la dérivée change de multiples fois pendant Δt_{DL}.

### II.3 Détermination de l'amplitude du flux buccal de l'utilisateur

La détermination de l'amplitude A_{buccal} du flux buccal F_{buccal} par les moyens 42 de traitement du signal délivré par l'organe 19 de mesure qualitative du débit, par exemple par la mesure de la résistance d'une thermistance, est analogue à celle de l'amplitude Aₙₐₛₐₗ pour le flux nasal Fₙₐₛₐₗ. En effet, après un certain lissage du signal brut et la soustraction d'un offset, on calcule la différence entre le maximum et le minimum du flux buccal F_{buccal}.

De préférence, pour réduire les étapes de calcul et de traitement, l'amplitude A_{buccal} n'est déterminée que lors d'une phase d'expiration nasale prolongée, c'est-à-dire ayant par exemple une durée supérieure à 3 secondes. Dans ce cas, c'est-à-dire à partir de l'instant t₁ représenté sur la figure 3, les moyens 42 reçoivent des moyens 29 un signal de commande pour calculer l'amplitude du flux buccal à chaque seconde, c'est-à-dire pour calculer la différence entre le flux buccal déterminé à un instant donnée et celui à un instant lui précédent par exemple d'une seconde.

### II.4 Détermination d'une phase de ronflement de l'utilisateur

Le ronflement est caractéristique d'un phénomène obstructif dans les voies respiratoires d'un utilisateur. Au niveau du signal de pression mesuré par le capteur 13, il se manifeste par des oscillations qui se superposent sur le signal normal de pression comme cela est représenté sur le graphique supérieur de la figure 6 montrant à titre d'exemple une courbe 90 représentative d'un ronflement.

Pour déterminer si l'utilisateur 3 souffre d'un ronflement, on réalise d'abord dans les moyens 45 un filtrage passe-haut du signal de pression et on amplifie le signal filtré dans les moyens 47. Le signal filtré et amplifié est représenté en fonction du temps par la courbe 92 sur le graphique inférieur de la figure 6.

Puis, on interpole par l'intermédiaire des moyens 49 le signal filtré amplifié pour obtenir une courbe-enveloppe 94. Cette courbe-enveloppe 94 passe par tous les maxima du signal filtré et amplifié et, dans le cas d'un ronflement, présente une forme caractéristique. C'est pourquoi cette courbe-enveloppe 94 est ensuite comparée dans les moyens 53 à la courbe de référence enregistrée dans les moyens 51 de mémorisation afin de déterminer la présence de phases de ronflement.

### II.5 Conversion des variables physiques traitées en des états de variables floues avec des degrés d'appartenance

A titre d'exemple, on explique ci-après en détail la conversion d'une variable physique traitée, l'amplitude du flux nasal Aₙₐₛₐₗ lorsque la durée de la phase d'expiration nasale est supérieure à trois secondes, pour établir son degré d'appartenance à un ou plusieurs états d'une variable floue associée, appelée A^{*f*}ₙₐₛₐₗ. Cette procédure de conversion s'applique de manière analogue à toutes les autres variables physiques à prendre en compte pour l'application de règles selon la logique floue.

Sur la figure 7 est représenté un graphique montrant en abscisses l'univers de discours du flux nasal Aₙₐₛₐₗ et en ordonnées les valeurs d'appartenance d'états de la variable floue A^{*f*}ₙₐₛₐₗ associée.

Comme on le voit sur la figure, la variable floue A^{*f*}ₙₐₛₐₗ peut prendre quatre états, à savoir les états appelés "faible", "moyen-faible", "moyen-fort" et "fort".

A chaque état de A^{*f*}ₙₐₛₐₗ est associée une courbe continue 100, 102, 104 et 106 permettant d'établir le degré d'appartenance d'une valeur de Aₙₐₛₐₗ à un ou plusieurs états de la variable floue A^{*f*}ₙₐₛₐₗ

Ainsi par exemple, la courbe 100 est associée à l'état "faible" et présenté la forme d'un plateau suivi d'une pente négative. La courbe 102 est associée à l'état "moyen-faible" et présente une forme de trapèze. La courbe 104 est associée à l'état "moyen-fort" et présente une forme triangulaire, et la courbe 106 est associée à l'état "fort" et présente une pente positive suivie d'un plateau.

La forme des courbes 100, 102, 104 et 106 est définie de façon empirique d'après des essais cliniques sur des utilisateurs. On note que les plateaux aux extrémités de l'univers de discours des variables physiques sont généralement retenus.

En outre, les valeurs en ordonnées des courbes 100, 102, 104 et 106 sont comprises entre zéro (0) et un (1).

Par ailleurs, il est important à noter que les diverses courbes 100, 102, 104 et 106 se chevauchent de sorte qu'une valeur de Aₙₐₛₐₗ peut appartenir à deux états de A^{*f*}ₙₐₛₐₗ.

Ainsi par exemple la valeur Aₙₐₛₐₗ= 0,1 appartient à
- un état "faible" de la variable floue A^{*f*}ₙₐₛₐₗ avec un degré d'appartenance de 0,1 et
- un état "moyen-faible" de la variable floue A^{*f*}ₙₐₛₐₗ avec un degré d'appartenance de 0,9.
Ceci est également représenté sur la figure 7.

Les autres variables physiques sont converties par les moyens 55 de conversion selon le même principe.

### II.6 Application de règles aux états de variables floues avec des degrés d'appartenance

Les états avec leurs degrés d'appartenance respectifs des variables floues sont introduits dans des moyens 23 d'application de règles mémorisées dans la mémoire 25 formant base de connaissances. Ces règles sont définies de façon empirique à partir d'essais cliniques sur des utilisateurs.

Chaque règle fait intervenir au moins deux variables floues différentes pour obtenir par exemple un degré d'appartenance à un état d'une phase de respiration du sommeil de l'utilisateur 3.

A titre d'exemple, on indique ci-après un jeu de onze règles pour deux variables floues, la variable floue susmentionnée A^{*f*}ₙₐₛₐₗ ainsi que la variable floue A^{*f*}_{buccal}. Cette variable floue A^{*f*}_{buccal} peut également prendre quatre états, à savoir les états appelés "faible", "moyen-faible", "moyen-fort" et "fort", et les degrés d'appartenance à ces états sont établis à partir de l'amplitude du flux buccal A_{buccal} lorsque la durée de la phase d'expiration est supérieure à trois secondes.

| N° | A^{*f*}_{buccal} | A^{*f*}ₙₐₛₐₗ | → | phase de respiration |
|---|---|---|---|---|
| 1 | moyen-fort | moyen-fort | | normal |
| 2 | faible | moyen-fort | | hypopnée |
| 3 | moyen-faible | moyen-fort | | hypopnée |
| 4 | moyen-fort | faible | | hypopnée |
| 5 | faible | faible | | apnée |
| 6 | moyen-faible | faible | | hypopnée |
| 7 | moyen-fort | moyen-faible | | hypopnée |
| 8 | faible | moyen-faible | | hypopnée |
| 9 | moyen-faible | moyen-faible | | hypopnée |
| 10 | fort | | | normal |
| 11 | | fort | | normal |

Supposons que Aₙₐₛₐₗ appartienne à
- un état "faible" de la variable floue A^{*f*}ₙₐₛₐₗ avec un degré d'appartenance de 0,1 et
- un état "moyen-faible" de la variable floue A^{*f*}ₙₐₛₐₗ avec un degré d'appartenance de 0,9,
et que A_{buccal} appartienne à
- un état "faible" de la variable floue A^{*f*}_{buccal} avec un degré d'appartenance de 0,7, et
- un état "moyen-faible" de la variable floue A^{*f*}_{buccal} avec un degré d'appartenance de 0,2.

Les moyens 23 appliquent les règles 1 à 11 de la manière suivante pour déterminer les phases de respiration selon la logique floue.

Tout d'abord, les moyens 23 ne considèrent que les règles concernées, c'est-à-dire les règles pour lesquelles un degré d'appartenance est affecté à un état de la première variable floue et un degré d'appartenance est affecté à un état de la seconde variable floue. Dans l'exemple présent, ce sont les règles N° 5, 6, 8 et 9.

Ensuite, on applique un ordre de sélection "MIN-MAX". Cet ordre consiste à affecter dans un premier temps à une phase respiratoire, associée à une règle déterminée, un degré d'appartenance égal au minimum des degrés d'appartenance des états desdites variables floues qui sont à considérer pour cette règle déterminée.

Dans l'exemple présent, en appliquant par exemple la règle N° 5, on obtient que le sommeil de l'utilisateur 3 est dans une phase respiratoire "apnée" avec un degré d'appartenance égal au MIN (0,7; 0,1) = 0,1.

De manière analogue, on obtient par l'application de la règle N°6 que le sommeil de l'utilisateur 3 est dans une phase respiratoire "hypopnée" avec un degré d'appartenance égal à MIN (0,2; 0,1) = 0,1, par l'application de la règle N° 8, dans une phase respiratoire "hypopnée" avec un degré d'appartenance égal à MIN (0,7; 0,9) = 0,7, et par l'application de la règle N° 9, dans une phase respiratoire "hypopnée" avec un degré d'appartenance égal à MIN (0,2; 0,9) = 0,2.

Comme résultat final des degrés d'appartenance des états respectifs des phases respiratoires du sommeil de l'utilisateur selon la logique floue, on considère dans un second temps le maximum d'appartenance obtenu pour chaque état, à savoir dans l'exemple présent, 0 pour l'état de respiration normale, 0,1 pour l'état de respiration d'apnée et 0,7 pour l'état de respiration d'hypopnée.

Ainsi, on comprend que l'utilisation de la logique floue permet d'affiner le diagnostic des phases respiratoires d'un utilisateur.

Avantageusement, les moyens 21 de traitement et de conversion ainsi que les moyens d'application 23 des règles et la mémoire 25 sont principalement réalisés par un ordinateur comportant des interfaces adaptées pour acquérir les signaux des capteurs 13, 17 et 19, et chargé de programmes adaptés pour traiter et exploiter ces signaux.

## Revendications

1. Dispositif de détermination de phases respiratoires du sommeil d'un utilisateur (3), comprenant des moyens (5) de mesure d'au moins deux variables physiques dont au moins une première variable physique est représentative du flux nasal de l'utilisateur (3) et dont au moins une seconde variable physique est représentative du flux buccal de celui-ci, caractérisé en ce qu'il comprend en outre des moyens (21) de traitement et de conversion de chaque variable physique en vue d'établir son degré d'appartenance à au moins un état d'une variable floue, et des moyens (23) d'application de règles préétablies entre au moins un état d'une première variable floue et un état d'une seconde variable floue pour évaluer le degré d'appartenance à un état de phase respiratoire du sommeil de l'utilisateur (3) selon la logique floue.

2. Dispositif selon la revendication 1, caractérisé en ce que chaque variable floue possède au moins deux états.

3. Dispositif selon l'une quelconque des revendications 1 ou 2, caractérisé en ce que les degrés d'appartenance des états associés à une variable floue sont établis à partir de courbes continues (100, 102, 104, 106) définies sur tout l'univers de discours d'une variable physique mesurée.

4. Dispositif selon l'une quelconque des revendications 1 à 3, caractérisé en ce que les moyens (5) de mesure comprennent un capteur de pression (13) relié à une pièce nasale (7) destinée à être portée par l'utilisateur (3) et en ce qu'une des variables physiques mesurées est le signal de pression mesuré par ledit capteur de pression (13).

5. Dispositif selon la revendication 4, caractérisé en ce que lesdits moyens (21) de traitement et de conversion comprennent des moyens (43) d'extraction du signal de pression mesuré de phases de ronflement associées à des phénomènes obstructifs de respiration.

6. Dispositif selon la revendication 5, caractérisé en ce que lesdits moyens (43) d'extraction de phases de ronflement comprennent des moyens (45) de filtrage passe-haut du signal de pression, des moyens (47) d'amplification du signal filtré, des moyens (49) d'interpolation dudit signal filtré amplifié pour obtenir une courbe-enveloppe, des moyens (51) de mémorisation d'une courbe de référence et des moyens (53) de comparaison de ladite courbe-enveloppe avec ladite courbe de référence pour déterminer la présence de phases de ronflement.

7. Dispositif selon l'une quelconque des revendications 1 à 6, caractérisé en ce que les moyens (5) de mesure comprennent un capteur de courant (17) mesurant le courant consommé par une turbine (9) à faible inertie qui est reliée à ladite pièce nasale (7), et en ce qu'une des variables physiques mesurées est le courant consommé par ladite turbine (9).

8. Dispositif selon la revendication 1, caractérisé en ce que les moyens (21) de traitement et de conversion comprennent des moyens (27) pour extraire du signal de courant consommé une image du flux nasal de l'utilisateur (3), des moyens (29) de détermination des phases d'inspiration et d'expiration nasales dudit utilisateur (3), des moyens (31) de calcul de la dérivée temporelle de l'amplitude du flux nasal lors d'une phase d'inspiration et des moyens (33) d'exploitation de la dérivée pour déterminer si l'utilisateur (3) souffre d'un phénomène obstructif partiel ou pas.

9. Dispositif selon la revendication 8, caractérisé en ce que lesdits moyens (33) d'exploitation de la dérivée de l'amplitude du flux nasal comprennent des moyens (35) de comparaison de la valeur absolue de la dérivée à au moins une valeur de référence (V_{R}), et des moyens (39) de mesure de la durée pendant laquelle la valeur absolue de la dérivée est inférieure à ladite au moins une valeur de référence (V_{R}).

10. Dispositif selon la revendication 9, caractérisé en ce que lesdits moyens (33) d'exploitation de la dérivée comprennent en outre des moyens (41) de relevé du nombre de changements de signe de la dérivée lorsque celle-ci est inférieure à la valeur de référence (V_{R}).

11. Dispositif selon l'une quelconque des revendications 1 à 10, caractérisé en ce que les moyens (5) de mesure comprennent un organe (19) de mesure de la résistance d'une thermistance destinée à être placée à proximité de la bouche dudit utilisateur (3), et en ce qu'une des variables physiques mesurées est la résistance mesurée par ledit organe de mesure (19) de la résistance de la thermistance.
